# EUROPEAN PATENT SPECIFICATION

(11) **EP 1 626 710 B1**
(45) Date of publication and mention of the grant of the patent: **01.10.2008**
(21) Application number: 04735084.8
(22) Date of filing: 27.05.2004
(51) Int. Cl.: A61K 31/16, A61P 25/18

(54) **USE OF 2,2,3,3, TETRAMETHYLCYCLOPROPANE CARBOXYLIC ACID DERIVATIVES FOR TREATING PSYCHIATRIC DISORDERS**
VERWENDUNG VON 2,2,3,3, TETRAMETHYLCYCLOPROPANE CARBONSÄURE DERIVATEN ZUR BEHANDLUNG VON PSYCHIATRISCHEN STÖRUNGEN
UTILISATION DE DERIVES D'ACIDE CARBOXYLIQUE 2, 2,3,3 TETRAMETHYLCYCLOPROPANE POUR LE TRAITEMENT DE TROUBLES PSYCHIATRIQUES

(30) Priority: 29.05.2003 IL 156203
(43) Date of publication of application: 22.02.2006
(73) Proprietor: YISSUM RESEARCH DEVELOPMENT COMPANY OF THE HEBREW UNIVERSITY OF JERUSALEM, Givat Ram, Jerusalem 91390 (IL); Ben Gurion University Of The Negev Research And Development Authority, 84105 Beer Sheva (IL)
(72) Inventor: BIALER, Meir, 96222 Jerusalem (IL); YAGEN, Boris, 93707 Jerusalem (IL); BELMAKER, Haim, 84965 Omer (IL); AGAM, Galila, 84965 Omer (IL); SHALTIEL, Galit, 84965 Omer (IL)
(74) Representative: Grünecker, Kinkeldey, Stockmair & Schwanhäusser Anwaltssozietät
(86) International application number: PCT/IL2004/000455
(87) International publication number: WO 2004/105746

(56) References cited:
- WO-A-95/09835
- WO-A-95/21814
- WO-A-03/064374
- US-A- 5 880 157
- US-A1- 2002 115 718
- ISOHERRANEN, NINA ET AL: "New CNS-active drugs which are second-generation valproic acid: can they lead to the development of a magic bullet?" CURRENT OPINION IN NEUROLOGY , 16(2), 203-211 CODEN: CONEEX; ISSN: 1350-7540, 2003, XP008035486
- BLOTNIK, SIMCHA ET AL: "Disposition of two tetramethylcyclopropane analogs of valpromide in the brain, liver, plasma and urine of rats" EUROPEAN JOURNAL OF PHARMACEUTICAL SCIENCES , 6(2), 93-98 CODEN: EPSCED; ISSN: 0928-0987, 1998, XP008035503
- BIALER M ET AL: "Pharmacokinetics analysis and antiepileptic activity of tetra-methylcyclopropane analogues of valpromide" PHARMACEUTICAL RESEARCH, NEW YORK, NY, US, vol. 13, no. 2, 1996, pages 284-289, XP009028319 ISSN: 0724-8741
- AGAM G ET AL: "Myo-inositol-1-phosphate (MIP) synthase: A possible new target for antibipolar drugs" BIPOLAR DISORDERS 2002 DENMARK, vol. 4, no. SUPPL. 1, 2002, pages 15-20, XP008035523 ISSN: 1398-5647
- GREENBERG M L ET AL: "Inhibition of myo-inositol-phosphate synthase by valproate; a mechanism for mood stabilization?" EUROPEAN NEUROPSYCHOPHARMACOLOGY, vol. 13, no. Supplement 4, September 2003 (2003-09), pages S107-S108, XP008035506 & 16TH CONGRESS OF THE EUROPEAN COLLEGE OF NEUROPSYCHOPHARMACOLOGY; PRAGUE, CZECH REPUBLIC; SEPTEMBER 20-24, 2003 ISSN: 0924-977X

## Description

### FIELD OF THE INVENTION

The present invention relates to the treatment of bipolar disorders.

### BACKGROUND OF THE INVENTION

***Bipolar disorder*.** Bipolar disorder, or manic-depressive illness, is a common condition with a life-time prevalence of 1-2% [Weissman, M. M., Leaf, P. J., Tischler, G. L., Blazer, D. G., Karno, M., Bruce, M. L., Florio, L. P. 1988. Affective disorders in five United States communities. Psychol Med 18:141-53]. Its episodic course with intervening full recovery belies the severe impact of this disorder. The cumulative effects of recurring bouts of mania and depression lead to an increased rate of marital and family breakdown, unemployment, impaired career progress and consequent financial difficulties. About 15% of bipolar patients commit suicide, and mortality rates due to physical disorders are also increased [Angst J, Clayton P. Premorbid personality of depressive, bipolar, and schizophrenic patents with special reference to suicidal issues. Compr Psychiatry 1986;27:511-32; Dilsaver SC. Change in the meaning of diagnostic concepts in psychiatry. J Clin Psychiatry 1987;9:152-162]. The effect on the broader community is highlighted by one estimation that the use of lithium saved the United States $4 billion in the period of 1969-1979, by reducing associated medical costs and restoring productivity [Reifman A, Wyatt RJ. Lithium: a brake in the rising cost of mental illness, Arch Gen Psychiatry 1980;37:385-388].
The discovery of lithium's efficacy as a mood-stabilizing agent revolutionized the treatment of patients with bipolar disorder. After five decades, lithium continues to be a mainstay of treatment, both for the acute manic phase, and as prophylaxis for recurrent manic and depressive episodes [Goodwin FK, Jamison KR. Manic-depressive illness New York: Oxford University Press, 1990]. However, despite its role as one of psychiatry's most important treatments, the biochemical basis for lithium's mood-stabilizing actions remains to be fully elucidated [Manji HK, Lenox RH. Lithium: a molecular transducer of mood-stabilization in the treatment of bipolar disorder. Neuropsychopharmacology 1998; 3: 161-166]. Furthermore, increasing evidence suggests that a significant number of patients respond poorly to lithium; 20-40% of patients fail to show an adequate antimanic response to lithium, while many others are helped but continue to suffer significant morbidity [Kramlinger KG, Post RM. The addition of lithium to carbamazepine. Antidepressant efficacy in treatment-resistant depression. Arch Gen Psychiatry 1989;46:794-800; Chou J C. Recent advances in treatment of acute mania. J Clin Psychopharmacol 1991; 11:3-21]. More recently, the branched chain fatty acid - valproic acid (VPA) has been used for treatment of bipolar disorder [Bowden CL, Brugger AM, Swann AC, Calabrese JR,Janicak PG, Petty F, Dilsaver SC, Davis JM, Rush AJ, Small JG, et al. Efficacy of divalproex vs lithium and placebo in the treatment of mania. The Depakote Mania Study Group. JAMA 1994; 271:918-924]. Like lithium, it is not completely effective. Although there are numerous biochemical and cellular effects of lithium and valproate their relevance to a therapeutic mechanism of action has not been elucidated, but the role of inositol metabolism has gained substantial support.

***Inositol biosynthesis*.** Inositol (I) is synthesized *de novo* in two steps. The first step, the conversion of glucose-6-phosphate (glucose-6-P) to inositol-1-phosphate (I-1-P), is catalyzed by *myo*-inositol-1-phosphate (MIP) synthase. The second step of the pathway is the conversion of I-1-P to inositol by *myo-*inositol monophosphatase (IMPase). This step is common to the production of inositol via the *de novo* synthetic pathway and its recycling in the phosphatidylinositol (PI) cycle.

### The effects of lithium and valproate (VPA)

There is increasing evidence that inositol may play an important role in bipolar disorders.

Biochemical studies have shown that lithium inhibits IMPase uncompetitively [Hallcher LM, Sherman W R. The effects of lithium ion and other agents on the activity of myo-inositol-1-phosphatase from bovine brain. J Biol Chem 1980; 255: 10896-10901; Atack JR, Broughton HB, Pollack SJ. Structure and mechanism of inositol monophosphatase. FEBS Lett 1995;361:1-7). Based on the observed uncompetitive inhibition by lithium, resulting in decreased inositol levels, an increase in inositol phosphates, and subsequent down regulation of the phosphoinositide cycle, Hallcher and Sherman [Hallcher LM, Sherman WR. The effects of lithium ion and other agents on the activity of myo-inositol-1-phosphatase from bovine brain. J Biol Chem 1980; 255: 10896-10901] and Berridge [Berridge M. Phosphoinositides and signal transduction. Rev Clin Basic Pharm 1985; 5 Suppl: 5-13] proposed *the inositol depletion hypothesis* according which lithium acts by depletion of inositol from the brain, resulting in dampening of an overstimulated PI cycle in patients. Support for this hypothesis comes from evidence that chronic lithium administration reduces agonist-stimulated phosphoinositide hydrolysis in rat brain [Kendall DA, Nahorski SR. Acute and chronic lithium treatments influence agonist and depolarization-stimulated inositol phospholipid hydrolysis in rat cerebral cortex. J Pharmacol Exp Ther 1987;241:1023-7; Casebolt, T. L., Jope, R. S. Long-term lithium treatment selectively reduces receptor-coupled inositol phospholipid hydrolysis in rat brain. Biol Psychiatry 1989;25:329-40; Godfrey PP, McClue SJ, White AM, Wood AJ, Grahame-Smith DG. Subacute and chronic in vivo lithium treatment inhibits agonist- and sodium fluoride-stimulated inositol phosphate production in rat cortex. J Neurochem 1989; 52:498-506]. It has recently been reported that VPA, like lithium, causes inositol depletion in yeast [Vaden DL, Ding D, Peterson B, Greenberg ML. Lithium and valproate decrease inositol mass and increase expression of the yeast INO1 and IN02 genes for inositol biosynthesis. J Biol Chem 2001;276(18):15466-71], *Dictyostelium* [Williams RS, Cheng L, Mudge AW, Harwood AJ. A common mechanism of action for three mood-stabilizing drugs. Nature 2002;417(6886):292-5] and mammals [Williams RS, Cheng L, Mudge AW, Harwood AJ. A common mechanism of action for three mood-stabilizing drugs. Nature 2002;417(6886):292-5; O'Donnell T, Rotzinger S, Nakashima T T, Hanstock C C, Ulrich M , Silverstone PH. Chronic lithium and sodium valproate both decrease the concentration of myo-inositol and increase the concentration of inositol monophosphates in rat brain. Brain Res 2000;880(1-2):84-91]. In addition, it has recently been also found that lithium, VPA and carbamazepine all cause increased spreading of the growth cones of newborn rat dorsal root ganglia (DRG) cells. These effects are reversed by addition of *myo*-inositol supporting the notion of an inositol depletion mechanism [Williams RS, Cheng L, Mudge AW, Harwood AJ. A common mechanism of action for three mood-stabilizing drugs. Nature 2002;417(6886):292-5].

Various attempts were made to discover new agents for treating bipolar disorders.

U.S. patent No. 6,555,585 discloses a method for the treatment of mania in bipolar disorder using derivatives of VPA and 2-valproenic acid. The antimanic effect of the compounds was evaluated using the amphetamine-induced hyperactivity model. This model focuses on an induced increase in the activity level of the animal as parallel to the hyperactivity in the manic patient. The reversal of the induced hyperactivity in rodents, by pretreatment with a drug indicates the possible efficacy of the drug in the treatment of human mania. The compounds disclosed in U.S. patent No. 6,555,585 are disadvantageous since they were found to be effective mainly for treating the manic phase in bipolar disease.

Moreover the use of VPA, - a broad spectrum antiepileptic drug useful also for treating bipolar disorders (both mania and depression) is limited by its considerable adverse effects including hepatotoxicity and teratogenicity and thus cannot be given to women of childbearing age and children [Baille, T. A. et al. In Antiepileptic Drugs, eds. R.H. Levy et al. Raven Press, New York. Pp. 641-651 (1989)].

There is thus a widely recognized need for, and it would be highly advantageous to have, new agents for treating bipolar disorders (both mania and depression) devoid of the above limitations.

U.S. patent No. 5,880,157 discloses derivatives of 2,2,3,3 tetramethylcyclopropane carboxylic acid for treating epilepsy. Isoherranen N. et al 2002 studied the anticonvulsant activity of N-methyl-tetramethylcyclopropyl carboxamide (M-TMCD) and its metabolite in various animal (rodent) models of human epilepsy, and evaluated their ability to induce neural tube defects (NTDs) and neurotoxicity. [Isoherranen N. et al. Anticonvulsant profile and teratogenicity of N-methyl-tetramethylcyclopropyl carboxamide; A new antiepileptic drug. Epilepsia 2002; 43:115 - 126]. M-TMCD (a cyclopropyl analog of VPA) was found to be advantageous compared to VPA because of its better potency as an anticonvulsant drug, its wider safety of margin, its lack of teratogenicity and its potential lack of hepatotoxicity.

### SUMMARY OF THE INVENTION

The present invention relates to the use of a compound of formula I 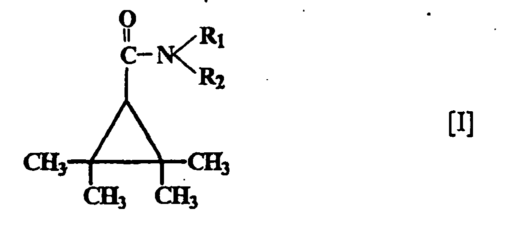
wherein R₁ and R₂ are different for the preparation of a medicament for treating bipoler disorders,
where, one of R₁ or R₂ is C₁-C₆ alkyl group and the other is hydrogen.

Additionally according to a preferred embodiment of the present invention, the C₁-C₆ alkyl group is a straight or a branched alkyl group.

Further according to a preferred embodiment of the present invention, the C₁-C₆ alkyl group is a methyl group.

Additionally according to a preferred embodiment of the present invention, the compound is administered as a pharmaceutical composition comprising a compound of formula I and a pharmaceutical acceptable carrier.

Moreover according to a preferred embodiment of the present invention, the route of administration of the compound is selected from the group consisting of oral, parenteral, topical, transdermal, rectal and buccal administration.

Further according to a preferred embodiment of the present invention, the route of administration of the compound is selected from the group consisting of oral and parenteral administration.

Still further according to a preferred embodiment of the present invention, the parenteral route of administration is selected from the group consisting of intravenous, intramuscular, intraperitoneal and subcutaneous administration.

Additionally according to a preferred embodiment of the present invention, the compound is administered in the range of from 1 mg to 1000 mg per day.

Moreover according to a preferred embodiment of the present invention, the compound is administered in the range of from 20 mg to 500 mg per day.

The invention also relates to a compound of formula I 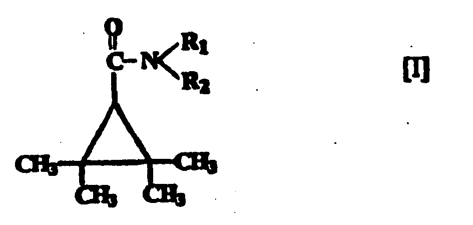 wherein R₁ and R₂ are different and one of R₁ or R₂ is a C₁-C₆ alkyl group and the other is hydrogen, for use in treating bipolar disorders.

The present invention further relates to a pharmaceutical composition for treating bipolar disorders comprising a pharmaceutically acceptable carrier and as an active ingredient a therapeutically effective amount of a compound of formula I 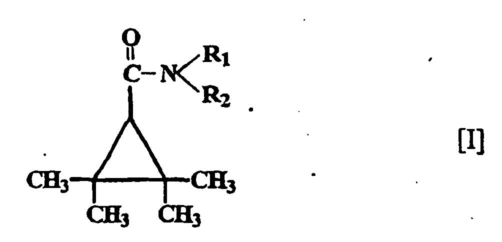 as defined above.

### DEFINITIONS

As used herein, the term M-TMCD refers equally to N-methyl-2,2,3,3-tetramethylcyclopropanecarboxamide; N-methyl-tetramethylcyclopropane carboxamide; and
N-methyl-tetramethylcyclopropyl carboxamide.

### BRIEF DESCRIPTION OF THE DRAWINGS

A more complete understanding of the invention and many of its advantages will become apparent by reference to the detailed description which follows when considered in conjunction with the accompanying figures wherein:
**FIG. 1** illustrates the frontal cortex inositol levels in mice injected with a single dose of VPA. Results are means ± S.E.M. ANOVA: F=3.14, df _{3,59}, p=0.031
   *Post hoc LSD tests: control vs. 300mg/kg VPA, p=0.038; control vs. 600mg/kg VPA, p=0.018; control vs. 800mg/kg VPA, p=0.024. W.W.= wet weight;
**FIG. 2** illustrates the *in-vitro* effect of VPA on MIP synthase activity in human brain homogenate (Dixon's plot). Results are means + S.E.M.
   The intersect of the best fit line with the X axis = -Ki
   n = 10 (0 mM), 5 (0.35 mM), 7 (0.525 mM), 7 (0.7 mM) and 3 (1.4 mM).
   MIP = myo-inositol-1-phosphate; VPA = valproic acid, V₀ = activity in the absence of VPA, V_{VPA} = activity in the presence of the appropriate VPA concentration;
**FIG. 3** illustrates a noncompetitive mode of inhibition of MIP synthase by VPA (A Lineweaver-Burk plot). Each point represents the mean + S.E.M. of 2-3 replicates. MIP = myo-inositol-1-phosphate; VPA = valproic acid.
   The intersect of the best fit line with the X axis = -1/Km.
   The intersect of the best fit line with the Y axis = 1/Vmax.
**FIG. 4** illustrates the effect of valproic acid (VPA), valpromide (VPD) and M-TMCD on spreading of rat neuron DRGs.
   a. control, b. 1 mM VPA, c. 1 mM VPD, d. 1 mM M-TMCD. Histograms show frequency distribution of the area of the growth cones.
   Units of the Y-axis are %
   Units of the X-axis are µm² of the spread area
   Gray bars = contracted growth cones (spread area < 50 micro m²).

### DETAILED DESCRIPTION OF THE INVENTION

The present invention discloses the use of 2,2,3,3-tetramethylcyclopropane carboxylic acid derivative compounds (compounds of formula 1) for treating bipolar disorder.

The present invention provides use of a compound of formula I 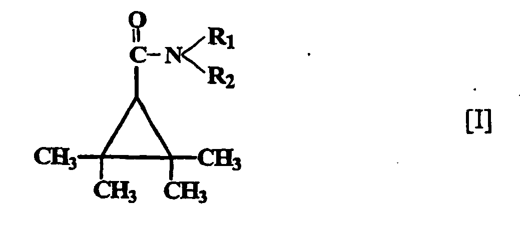 wherein R₁ and R₂ are different and one of R₁ or R₂ is hydrogen and the other is a C₁-C₆ alkyl group, for the preparation of a medicament for treating a bipolar disorder.

As used herein the term "treating" includes prophylactic and/or therapeutic uses and refers to abrogating, preventing, alleviating, slowing or reversing the progression of a disease or disorder, or substantially preventing the appearance of clinical symptoms of a disease or disorder.

As used herein the term "therapeutically effective amount "refers to an amount of a compound sufficient to bring about at least one of the effects defined under the term treating.

In compounds of structure formula I.

Preferably the C₁-C₆ alkyl group is a C₁-C₄ alkyl group and most preferably the C₁-C₆ alkyl group is a methyl group.

Most preferred compound is N-methyl-2,2,3,3-tetramethylcyclopropanecarboxamide (M-TMCD). (Most preferably in formula I, one of R₁ or R₂ is a methyl group and the other is hydrogen). The C₁-C₆ alkyl group may be a straight or a branched alkyl group. The C₁-C₄ alkyl group may be a straight or a branched alkyl group.

Whenever a numerical range e.g. "1-6" is stated herein, it means that the group in this case the alkyl group, may contain 1 carbon atom, 2 carbon atoms, 3 carbon atoms, etc., up to and including 6 carbon atoms.

The psychiatric disorder treated is a bipolar disorder. The compounds of the present invention have a parallel effect to mood stabilizers and are therefore expected to be useful for treating both the manic and depression phases of bipolar disorders.
The mammal may be a human.

Preferably the compound of formula I is administered as a pharmaceutical composition comprising a compound of formula I and a pharmaceutical acceptable carrier.

As used herein the term "pharmaceutically acceptable carrier" refers to an inert non-toxic carrier or diluent that does not cause significant irritation to a subject (mammal) and does not abrogate the biological activity and properties of the administered compound (active ingredient).
As used herein, the term "pharmaceutical acceptable carrier" encompasses any of the standard pharmaceutical accepted carriers such as lactose, sodium chloride, glucose, starch, calcium carbonate, kaolin, cellulose, lower alkyl ethers of cellulose, stearic acid, aluminum silicate, polyethylene glycols, cacao butter, higher alcohols, esters of higher alcohols, gelatin, semi-synthesized glycerides, water, alcohols, oils, fatty acids, liquid preparations, emulsion preparations and suspension preparations.

The "pharmaceutical acceptable carrier" may include any sustained release material known in the art such as polymers or waxes.

Additional excipients such as binders, disintegrants, adsorbents, lubricants, wetting agents, buffering agents, isotonic agents, surface active agents, suspending agents and polymers may be added to the pharmaceutical compositions.

Moreover, if necessary, coloring agents, preservatives, flavoring agents, sweetening a gents and other ingredients may be added to the pharmaceutical compositions.

The pharmaceutical acceptable carrier may be a solid, a semi-solid, or a liquid material.

The pharmaceutically acceptable carrier selected depends on the final form of the composition.

The final form of the pharmacetical composition may be for example a liquid, an emulsion, a suspension, a solution, a syrup, an elixir, drops, a spray, a cream, an ointment, a lotion, a gel, a paste, an inhalant, a powder, a granule, a tablet, a caplet, a pill, a capsule, a lozenge, a pastille, a suppository, a transdermal patch or an injection.

The route of administration may be for example, oral, parenteral, topical, transdermal, rectal or buccal administration.

Preferably, the route of administration is oral or parenteral such as intravenous, intramuscular, intraperitoneal or subcutaneous and most preferably the route of administration is oral.

Compositions for administration by the oral route, in the form of for example tablets or capsules, are preferred.

Compositions for oral use such as tablets and capsules where the typical solid carrier is an inert substance such as lactose, starch, glucose, methyl cellulose, magnesium sterate, dicalcium phosphate, mannitol and the like, may be prepared by conventional means with pharmaceutically acceptable excipients such as binding agents (for example polyvinylpyrrolidone or hydroxypropyl m ethylcellulose); lubricants (for example magnesium stearate, talc or silica); disintegrants (for example sodium starch glycollate); or wetting agents (for example sodium lauryl sulphate). Tablets may be coated by methods well known in the art.

Liquid preparations for oral administration may take the form of, for example, solutions, syrups elixirs, emulsions or suspensions. For oral administration in a liquid form, a compound of formula I may be combined with any oral, pharmaceutically acceptable carrier such as ethanol, glycerol, water, or a mixture thereof.

Moreover, such liquid preparations may be prepared by conventional means with pharmaceutically acceptable additives such as suspending agents (for example cellulose derivatives or hydrogenated edible fats); emulsifying agents (for example lecithin or acacia); non-aqueous vehicles (for example almond oil, oily esters, ethyl alcohol or fractionated vegetable oils); and preservatives (for example methyl or propyl-p-hydroxybenzoates). The preparations may also contain buffer salts, flavouring, colouring and sweetening agents as appropriate.

For parenteral administration the compositions may be in the form of a suspension, a solution or an emulsion in oily or aqueous vehicles, and may contain pharmaceutically acceptable additives such as suspending agents, emulsifying agents or dispersing agents.

Pharmaceutical compositions of the invention for rectal administration may be presented as a suppository, which may be prepared by mixing one or more compounds of the invention with a suitable carrier comprising for example, cocoa butter, polyethylene glycol or semi-synthesized glycerides.

Dosage forms for topical or transdermal administration include sprays, ointments, pastes, creams, lotions, gels, solutions, patches and inhalants. Transdermal patches have the added advantage of providing controlled delivery of the compound to the body. The transdermal compositions may be prepared by dissolving or dispersing the compound in a proper carrier (a liquid, a semi-solid or a solid carrier). The flux rate of the compound can be controlled by either providing a rate controlling membrane or dispersing the active compound in a polymer matrix or gel.

Pharmaceutical compositions of the invention for buccal administration are adapted for retention in the mouth rather than swallowing, and consequent release the active component in the buccal cavity. Buccal compositions may be in the form of tablets, lozenges or pastilles. Most usually, the composition will be chewed or sucked to lead to the release of the active compound in the mouth. It is also possible to use tablets, for example in the form of a disc of polymeric material, which are attached to the wall of the buccal cavity and which gradually release the compound without being sucked.

The compositions for buccal administration may include carriers such as sucrose, mannitol, sorbitol, acacia, tragacanth, gelatin, glycerin, lactose, calcium carbonate or magnesium phosphate.

Lozenges usually include the active compound in a flavoured base such as sucrose and acacia or tragacanth: Pastilles may include the compound in an inert base such as gelatin and glycerin or sucrose and acacia.

Adhering agents with low speed of dissolution such as methyl cellulose, hydroxypropyl cellulose, hydroxypropyl methyl cellulose, carboxy methyl cellulose or copolymers of methacrylic - and acrylic acid may be used in the preparation of buccal compositions. Additional excipients may be added, for example: binding agents such as polyvinyl pyrrolidone, sweetening agents such as calcium saccharinate, lubricating agent such as magnesium stearate, flavouring agents such as maltol, or vanillin.

The pharmaceutical compositions of the invention may be prepared by methods of pharmacy well known to those skilled in the art. See generally, Remington's Pharmaceutical Sciences, 16th ed., Mack Publishing Company, Easton, Pennsylvania. (1980).

In the practice of the invention the amount of the compound incorporated in the pharmaceutical composition may vary widely. Factors considered when determining the precise amount are well known to those skilled in the art. Examples of such factors include, but are not limited to, age, sex and weight of the subject being treated, severity of the disease, the dosage form, route of administration being employed and the frequency with which the composition is to be administered.

Preferably the therapeutically effective amount of the compound is in the range of from 1 mg to 1000 mg per day (preferably administered orally) and most preferably the therapeutically effective amount is in the range of from 20 mg to 500 mg per day.

For oral administration the therapeutically effective amount of the compound may be several times greater than for parenteral administration.

The daily dose may be administered either singly or in multiple dosage over 24-hour period.

The compounds of formula I can be combined with other anti psychiatric medicaments.

The present invention additionally provides a pharmaceutical composition for treating a bipolar disorder comprising a pharmaceutically acceptable carrier and as an active ingredient a therapeutically effective amount of a compound of formula I as described in the present invention.

The compounds of formula I 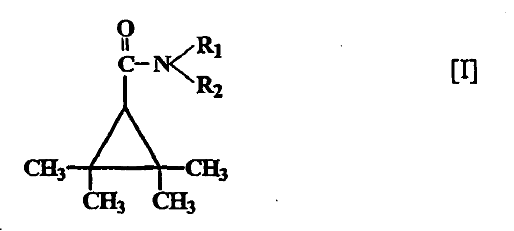 can be used, for the preparation of an inhibitor of MIP synthase.

Most preferred compound is
N-methyl-2,2,3,3-tetramethylcyclopropanecarboxamide (M-TMCD).

As used herein the term "effective amount" refers to an amount which inhibits MIP synthase in a manner which is statistically significant compared to control.

We have now found that human brain MIP synthase is specifically inhibited *in-vitro* by therapeutically-relevant VPA concentrations and that *in-vivo* VPA administration to mice reduces brain inositol levels.

It has been surprisingly found in the present invention that N-methyl-2,2,3,3-tetramethylcyclopropanecarboxamide (M-TMCD) inhibits MIP synthase activity by 93% while Valpromide (VPD), the amide of valproic acid - only by 34%. Consistent with this is the effect of M-TMCD and VPD on the growth cones of DRGs. VPD showed no increased growth cone spreading while M-TMCD had a similar effect to that of VPA.

M-TMCD is highly advantageous for treating bipolar disorders because of its high potency, and due to previous studies which have shown a wider safety of margin and lack of teratogenicity [U.S. pat No. 5,880,157; Isoherranen N. et al. Anticonvulsant profile and teratogenicity of N-methyl-tetramethylcyclopropyl carboxamide; A new antiepileptic drug. Epilepsia 2002; 43:115 - 126.]. M-TMCD is additionally advantageous due to its potential lack of hepatotoxicity. Therefore it can be given to women of childbearing age and children.

The following Experimental Details are set forth to aid in an understanding of the invention.

### EXAMPLES

### Synthesis of compounds

The compounds of formula I of the present invention can be prepared according to the methods and procedures described in Sterling et al. (U.S. patent No. 5,880,157), or variations thereof which will be apparent to those skilled in the art.
M-TMCD was prepared according t o the method disclosed in Sterling et al (U.S. patent No. 5,880,157).

### Experimental Examples

In the present study we tested the hypotheses that human brain MIP synthase is specifically inhibited *in-vitro* by therapeutically-relevant VPA concentrations and that *in-vivo* VPA administration to mice reduces brain inositol levels.

Evaluation of possible anti-bipolar effect (mood stabilizing effect) of M-TMCD was examined in the present study. The effect of M-TMCD on human brain MIP synthase activity was studied and compared to VPA and VPD.

The MIP synthase model is acceptable for examining bipolar disorders and developing antibipolar drugs [Agam G, Shamir A, Shaltiel G, Greenberg ML. Myo-inositol-1-phosphate (MIP) synthase: a possible new target for antibipolar drugs. Bipolar Disorder. 2002;4 Suppl 1:15-20].

### Example 1

### Experimental protocol

*Animals*. The study was approved by the Ben-Gurion University (Beer Sheva, Israel) Institutional Review Committee for the Use of Animals, and the procedures were carried out in compliance with the declaration of the National Institute of Health Guide for care and use of laboratory animals [Council NR, editor. Guide for the care and use of laboratory animals. Washington, D.C.: National Academy Press; 1996]. For acute VPA administration male ICR (Harlan) mice (20-25g) were divided into five groups (n=13 in each group). Two groups were injected intraperitonealy (IP) with a single dose of saline, and three groups- a single injection of different doses of VPA (300, 600, 800 mg/kg). 400 mg/kg/day VPA IP twice daily for 9 or 28 days resulted in blood VPA levels of 0.27 ± 0.02 mM [Chen G, Zeng WZ, Yuan PX, Huang LD, Jiang YM, Zhao ZH, et al. The mood-stabilizing agents lithium and valproate robustly increase the levels of the neuroprotective protein bcl-2 in the CNS. J Neurochem 1999;72(2):879-82] which approximate therapeutically-relevant plasma levels. Administration of higher V PA d oses i n mice t o attain similar plasma levels as in humans is required due to a ten times higher half-life time of VPA i n mice than in humans [Loscher W. V alproate: a reappraisal of its pharmacodynamic properties and mechanisms of action. Prog Neurobiol 1999;58(1):31-59]. One saline-injected group was sacrificed right after the injection. All other four groups were sacrificed one hour after the injection. Frontal cortex was removed from all mice right after sacrifice and frozen (-70°C) till further processing. For chronic VPA administration two independent experiments of two male ICR (Harlan) mice (20-25g) groups were carried out. Experiment 1: mice of group I (n=29) were treated with VPA in drinking water (12.5 g/liter) for 11 days. Mice of group II (n=30, controls) drank the same water without VPA. Experiment 2: Mice of group I (n=15) were injected twice a days (IP) increasing doses of VPA (400-800 mg/kg/day) for fourteen days. Mice of group II (n=13, controls) were injected saline (IP) twice a day. Animals were then sacrificed, brain removed and dealt as above.

*Gas chromatographic measurement of brain inositol levels.* Mouse brain free inositol levels were analyzed as trimethylsilyl (TMS) derivatives by gas-liquid chromatography (GC), as described in Shapiro J *et al* [Shapiro J, Belmaker RH, Beigon A, Seker A, Agam G. Scyllo-Inositol in Post-Moitem Brain of Suicide Victims, Bipolar, Unipolar and Schizophrenic Patients. J Neural T ransm 2000;107: 6 03-607] using a capillary column. The correlation coefficients of the daily standard curves were always above 0.987. Replicate reliability was tested in rat brain. When two different specimens from four rat brains were sampled, the correlation coefficient was 0.80. When a single brain extract was divided into ten separate samples, each assayed individually, the coefficient of variation was 23.5%. Each sample extract was assayed at least in duplicate. The results presented are the average of the replicates. Assays were performed in a blind and balanced design such that each run included samples from all comparison groups.

### Results

### Effect of acute and chronic VPA administration on mice brain inositol levels

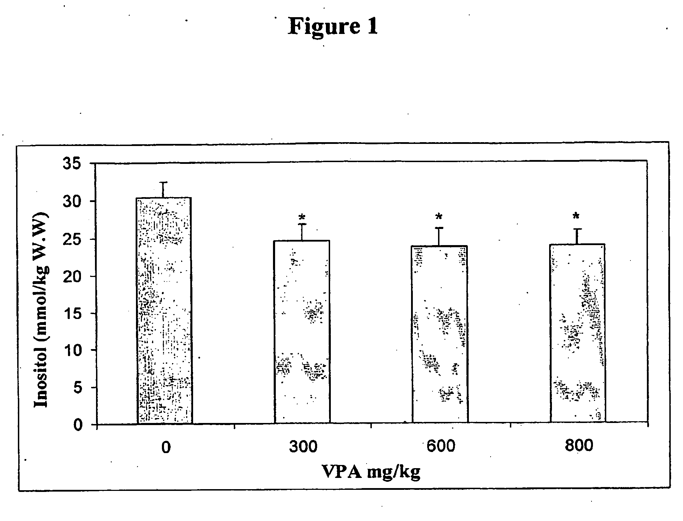

Gas chromatography was used to directly measure frontal cortex inositol levels in mice injected with a single dose of VPA. It was found that acute VPA administration to mice resulted in a statistically significant 20% reduction in frontal cortex inositol concentration at all concentrations tested (Figure 1). Since both control groups (animals sacrificed immediately after the saline injection or one hour after the injection) gave exactly the same mean inositol levels the two groups were pooled. Inositol levels of two mice (one from the control group and one from the 800 mg/kg VPA group) were excluded since they exceeded two S.D. of each group's mean.

In a similar observation to that seen with lithium, it was found that administration of VPA to mice for eleven days in drinking water or 14 days IP VPA injection did not deplete brain inositol levels (Table 1).

**Table 1. Chronic VPA administration does not affect mice whole brain inositol levels**

| **Treatment** | **Control** | **VPA** |
|---|---|---|
| **VPA in drinking water** | 32.5± 2.0 (n=30) | 30.1± 1.7 (n=29) |
| **IP injection of VPA** | 29.0± 2.7 (n=13) | 27.9± 3.2 (n=15) |

| | | |
|---|---|---|
| Results are means ± S.E.M. | | |

### Discussion

In the present study we found that acute but not chronic VPA administration to mice results in 20% reduction in frontal cortex inositol concentration. This suggests that both lithium and VPA have short term effects on brain inositol levels, but after long term treatment concentrations return to untreated levels. This may represent a long-term adaptation process, which readjusts inositol metabolism to restore the *myo*-inositol concentration. The acute decrease in intracellular inositol may further lead to a cascade of secondary changes associated with mood stabilization, such as altered gene expression.

Intracellular free inositol is supplied from three sources: specific uptake of *myo*-inositol originating in the diet; recycling of the inositol moiety from phosphoinositides by IMPase and *de-novo* synthesis from glucose-6-phosphate to form inositol-1-phosphate by MIP synthase and then dephosphorylation by IMPase [Fisher SK, Novak JE, Agranoff BW. Inositol and higher inositol phosphates in neural tissues: homeostasis, metabolism and functional significance. J Neurochem 2002;82(4):736-54].

In yeast, VPA reduces the concentration of *myo*-inositol, but without a build-up of inositol-1-phosphate. T his suggested t hat V PA may alter inositol synthesis rather than recycling it [Vaden DL, Ding D, Peterson B, Greenberg ML. Lithium and valproate decrease inositol mass and increase expression of the yeast INO1 and INO2 genes for inositol biosynthesis. J Biol Chem 2001;276(18):15466-71]. Indeed, we now show in the present invention that human brain MIP synthase activity is inhibitable by therapeutically-relevant VPA concentrations. Such an inhibition, in a similar manner to the inhibition of IMPase by lithium, leads, at the acute phase, to depletion of frontal cortex inositol. Thus, our results further elaborate Berridge's inositol depletion hypothesis [Berridge MJ. Phosphoinositides and signal transductions. Rev Clin Basic Pharm 1985;5 Suppl:5S-13S] concerning the first event in the molecular mechanism of antibipolar treatment suggesting a common mechanism for the two antibipolar drugs. Although acting through different targets they both induce the same effect of lowering frontal cortex intracellular inositol levels. Since VPA, in contrast with lithium, has been shown to have no effect on IMPase activity [Vadnal R, Parthasarathy R. Myo-inositol monophosphatase: diverse effects of lithium, carbamazepine, and valproate. Neuropsychopharmacology 1995;12(4):277-85], it is most probable that the acute *in-vivo* reduction of inositol levels following VPA treatment is a result of the direct inhibition of MIP synthase activity.

### Example 2

### Experimental protocol

*Human brain MIP synthase activity*. Five µl of the supernatant fraction obtained after sonication ( Ultrasonic Processor, N ewtown, C T, 15 sec at 0.1 output watts at 4°C) and centrifugation for 20 min at 9,000 X g at 4°C of postmortem human prefrontal cortex (1 mg wet weight in 0.5 ml of 50 mM Tris HCl, pH 7.4) were added to 20 µl reaction mixture containing 4mM D-glucose-6-phosphate, 1.25 µCi D-glucose-6-phosphate [¹⁴C], 1.6mM NAD⁺, 0.45 mM KCl, 5.4 mM MgCl₂ and 0.9 mM Tris HCl, pH 7.6, with or without 5 mU IMPase (Sigma, St. Louis). Incubation with or without various drugs concentrations (table 2 and table 3) was carried out for 3 hours (within the linear range) at 37°C. The reaction was stopped by adding 50 µl cold double distilled water (ddH₂0). Seventy out of the 80 µl were added to test tubes containing 1.25 g strong basic anion resin (Amberjet 4200, Rohm and Haas, Philadelphia) in 1 ml ddH₂0. The mixtures were vortexed for 10 min, then centrifugated for 10 min at 10,000 X g at room temperature, and 200 µl supernatant taken for ¹⁴C counting (Liquid Scintillation Counter, Kontron, Basel). The enzymatic activity was calculated by subtracting values without IMPase from the measures with IMPase. The measurements were carried out in triplicate.

Table 2 details the drugs and their concentrations compared with their therapeutic concentrations, studied for possible inhibition of human brain MIP synthase activity. Each drug was tested twice, each experiment in triplicate.

The use of the human brain collection was approved by our hospital Helsinki committee (IRB).

**Table 2. Psychotropic drugs screened for possible inhibition of MIP synthase**

| | | **Therapeutic range** | | **Concentration studied** | |
|---|---|---|---|---|---|
| **Type of Drug** | **Drug** | µ**g/ml** | **ng/ml** | µ**g/ml (mM)** | **ng/ml(µM)** |
| Anticonvulsant mood stabilizers | Carbamazepine | 4-12 | | 20 (0.08) | |
| | Phenytoin | 10-15 | | 20 (0.08) | |
| | Lamotrigine | 8-20 | | 30 (0.12) | |
| Typical antipsychotics | Chlorpromazine | | 25-150 | | 250 (0.78) |
| | Haloperidol | | 2-15 | | 30 (0.08) |
| Atypical antipsychotics | Clozapine | | ≥350 | | 750 (2.29) |
| Tricyclic antidepressants | Chlomipramine | | 200-500 | | 750 (2.63) |
| | Imipramine | | 50-350 | | 500 (1.57) |

**Rat neuron DRG (dorsal root ganglia) explants.** DRGs were dissected from the spinal cord area of newborn Sprague Dawley rats and cultured individually on poly-ornithine and laminin coated coverslips in serum-free medium supplemented with mouse 7S form nerve growth factor (NGF-7s, 25 ng/ml) at 37°C with 5% CO₂. After 24 hours for attachment, cytosine β-arabinofuranoside (ara-C, 10 µM) was added for 24 hours to kill non-neuronal cells. VPA, VPD and M-TMCD (1 mM) in fresh serum-free medium were then added to the DRG explants for 24 hour exposure. The explants were then fixed in 4% paraformaldehyde in PBS (Phosphate buffered saline) for 20 minutes at room temperature. Growth cones were, analysed on an inverted microscope (Zeiss Axiovert) and measured using an NIH Image software.

### Results

### VPA inhibits MIP synthase activity in human brain crude homogenate

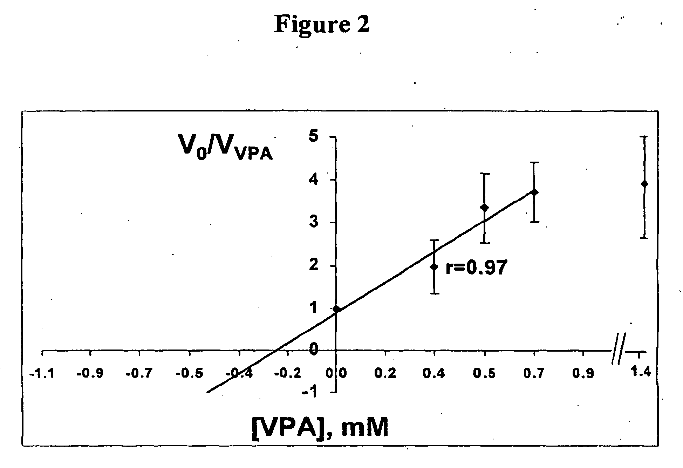

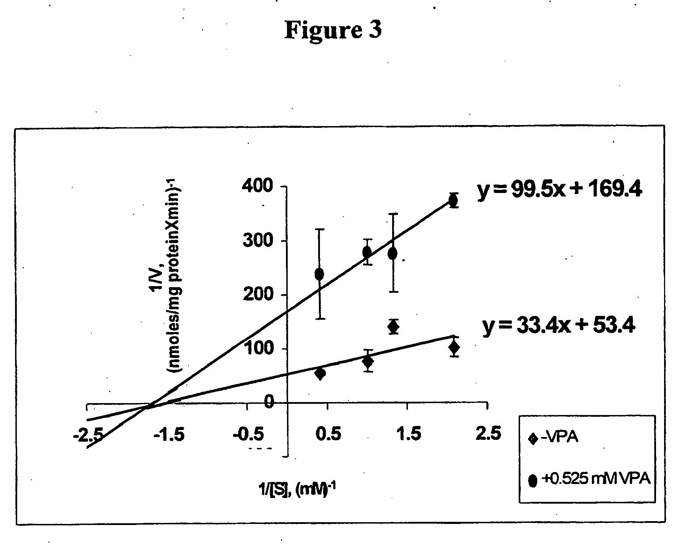

The *in-vitro* effect of VPA on MIP synthase activity in human brain homogenate was studied. A Dixon's plot (Figure 2) shows that human brain MIP synthase activity is inhibited by therapeutic concentrations of VPA (plasma range 0.35-0.7 mM) with a Ki of 0.21 mM. MIP synthase activity at substrate (glucose-6-phosphate) concentrations in the range of 0.48-2.48 mM in the presence and in the absence of 0.525 mM VPA was measured. The results presented as a Lineweaver-Burk plot reveal a noncompetitive mode of inhibition of MIP synthase by VPA (Figure 3). The Km of MIP synthase for glucose-6-phosphate and the Vmax derived from the figure are 0.625 mM and 0.02 nmoles/min X mg protein, respectively. The Km in the presence of 0.525 mM VPA does not change and the Vmax decreases to 0.006 nmoles/min X mg protein.

Km and Vmax are Michaelis Menten constants. See Christopher K. Mathews and K.E. Van Holde. Biochemistry. The Benjamin/cummings Publishing Company, Inc. 1990 (357-362).

### MIP synthase inhibition by VPA, VPD and M-TMCD correlates with their effect on rat neurons

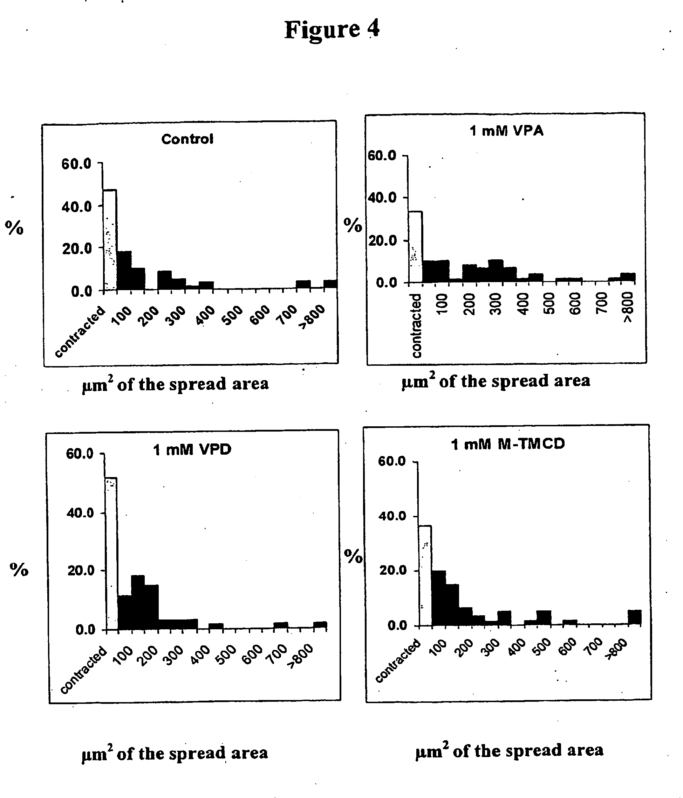

It was surprisingly found that at comparable concentrations M-TMCD inhibited MIP synthase activity by 93% while VPD - only by 34% (Table 3). Consistent with this is the effect of VPD and M-TMCD on the growth cones of DRGs. VPD showed no increased growth cone spreading while M-TMCD had a similar effect to that of VPA (Figure 4).

**Table 3. Effect of VPD and M-TMCD on human brain MIP synthase activity**

| **Treatment** | **Inhibition of human brain MIP synthase activity** |
|---|---|
| Control | 0% |
| VPA (1.0 mM) | 100% |
| VPD (1.4 mM) | 34% |
| M-TMCD (1.3 mM) | 93% |

| | |
|---|---|
| n = 10 (control), 10 (VPA), 3 (VPD), 2 (M-TMCD) | |

### Specificity of human brain MIP synthase inhibition by VPA and M-TMCD

We examined the effect of other psychotropic drugs on human brain MIP synthase activity. None of the other anticonvulsant mood stabilizers, carbamazepine, lamotrigine or phenytoin; the typical antipsychotics, haloperidol and chlorpromazine; the atypical antipsychotic, clozapine or the tricyclic antidepressants, chlomipramine and imipramine, at concentrations 1.5 times greater than their upper therapeutic range (Table 1), inhibited MIP synthase activity.

### Discussion

Glucose-6-phosphate is the substrate of MIP synthase. O'Donnell et al. [O'Donnell T, Rotzinger S, Nakashima TT, Hanstock CC, Ulrich M, Silverstone PH. Chronic lithium and sodium valproate both decrease the concentration of myo-inositol and increase the concentration of inositol monophosphates in rat brain. Brain Res 2000;880(1-2):84-91] measured a basal glucose-6-phosphate concentration of 0.226 µmol/g in rat brain. Assuming similar brain glucose-6-phosphate levels in human and rat, *in-vivo* MIP synthase activity is below half of its Vmax in the human brain, enabling a significant range for regulatory manipulation. The range of 0.02-0.2 nmoles/min X mg protein of human brain MIP synthase Vmax obtained from all control experiments (no drug added) in the present study is comparable with the Vmax found by Barnett et al. [Barnett JE, Brice RE, Corina DL. A colorimetric determination of inositol monophosphates as an assay for D-glucose 6-phosphate-1L-myoinositol 1-phosphate cyclase. Biochem J 1970;119(2):183-6] in rat testis, 0.104 nmoles/min X mg protein, and differs from that of Eisenberg et al. [Eisenberg F, Jr. D-myoinositol 1-phosphate as product of cyclization of glucose 6-phosphate and substrate for a specific phosphatase in rat testis. J Biol Chem 1967;242(7):1375-82].

The values obtained for human brain MIP synthase Ki for VPA, 0.21 mM from the Dixon's plot and 0.24 mM from the Linweaver-Burk plot are in good agreement. Since drug levels of VPA in brain are about 2 0% of blood levels [Loscher W. Valproate: a reappraisal of its pharmacodynamic properties and mechanisms of action. Prog Neurobiol 1999;58(1):31-59], therapeutic plasma levels of VPA would be sufficient for about 50% inhibition of MIP synthase activity in this tissue. The noncompetitive mode of inhibition of MIP synthase by VPA suggests that VPA interacts with a region distinct from the catalytic site of the enzyme and argues against the speculation of Stein and Geiger [Stein AJ, Geiger JH. The crystal structure and mechanism of 1-L-myoinositol- 1-phosphate synthase. J Biol Chem 2002;277(11):9484-91] based on their study of the yeast enzyme three-dimensional structure, that VPA targets the hydrophobic surface of the substrate binding cavity visualized in their study. In human brain tissue and neuronal cell cultures, the three pathways that supply intracellular free inositol exhibit the following comparable Vmax values: for uptake via the sodium-*myo*-inositol transporter (SMIT) - 0.06-0.10 nmoles/min x mg protein [Hertz L, Wolfson M, Hertz E, Agam G, Richards M, Belmaker RH. Lithium-inositol interactions: synthesis, uptake, turnover. In: Honing A, Van Praag HM, editors. Depression: neurobiological, psychopathological and therapeutic advances. chichester: Weily; 1997. p. 519-534] and for inositol-1-phosphate dephosphoryalation via IMPase - 0.2-2.0 nmoles/min x mg protein [Shaltiel G, Shamir A, Nemanov L, Yaroslavsky Y, Nemets B, Ebstein RP, et al. Inositol Monophosphatase Activity in Brain and Lymphocyte-derived Cell Lines of Bipolar, Schizophrenic and Unipolar Patients. World J Biol Psychiatry 2001;2:95-8]. This makes the calculated value of 0.02-0.20 n moles/min x mg protein for M IP s ynthase a meaningful contribution to cellular brain *myo*-inositol.

We now show that the inhibition of the human brain enzyme is specific for VPA and M-TMCD and cannot be demonstrated by other antibipolar drugs, typical antipsychotics, atypical antipsychotics and tricyclic antidepressants.

Intriguingly, the inhibitory effect VPD and M-TMCD on human brain MIP synthase activity also correlates with their effect on the growth cones of rat DRGs.

We showed that therapeutically relevant concentrations of VPA reduce brain *myo*-inositol levels. However, we have discovered that the target of VPA and, similarly, M-TMCD is distinct from that of lithium. Lithium inhibits IMPase thus affecting both recycling and *de-novo* synthesis of inositol, whereas the effect of VPA and M-TMCD is limited to the de-novo synthesis.

The results of the present study indicate that M-TMCD is preferred compared to VPA and VPD for treating bipolar disorders because of its high potency and due to previous studies which have shown wider safety of margin and lack of teratogenicity [U.S. pat No. 5,880,157; Isoherranen N. et al. Anticonvulsant profile and teratogenicity of N-methyl-tetramethylcyclopropyl carboxamide; A new antiepileptic drug. Epilepsia 2002; 43:115 - 126]. M-TMCD is additionally advantageous due to its potential lack of hepatotoxicity. Therefore it can be given to women of childbearing age and children.

Although the invention has been described in conjunction with specific embodiments thereof, it is evident that many alternatives, modifications and variation will be apparent to those skilled in the art. Accordingly, it is intended to embrace all such alternatives, modifications and variations that fall within the spirit and broad scope of the appended claims.

## Claims

1. A compound of formula I 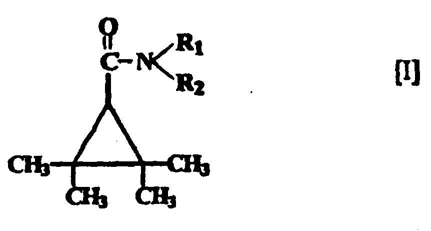 wherein R₁ and R₂ are different and one of R₁ or R₂ is a C₁-C₆ alkyl group and the other is hydrogen, for use in treating bipolar disorders.

2. The compound of formula I according to claim 1 wherein said C₁-C₆ alkyl group is a straight or a branched alkyl group.

3. The compound of formula I according to claim 1 wherein said C₁-C₆ alkyl group is a methyl group.

4. The compound of formula I according to claim 1 wherein said compound is administered as a pharmaceutical composition comprising a compound of formula I and a pharmaceutical acceptable carrier.

5. The compound of formula I according to claim 1 wherein the route of administration of said compound is selected from the group consisting of oral, parenteral, topical, transdermal, rectal and buccal administration.

6. The compound of formula I according to claim 1 wherein the route of administration of said compound is selected from the group consisting of oral and parenteral administration.

7. The compound of formula I according to claim 5 or 6 wherein said parenteral route of administration is selected from the group consisting of intravenous, intramuscular, intraperitoneal and subcutaneous administration.

8. The compound of formula I according to claim 1 wherein said compound is administered in the range of from 1 mg to 1000 mg per day.

9. The compound of formula I according to claim 1 wherein said compound is administered in the range of from 20 mg to 500 mg per day.

## Patentansprüche

1. Verbindung der Formel I 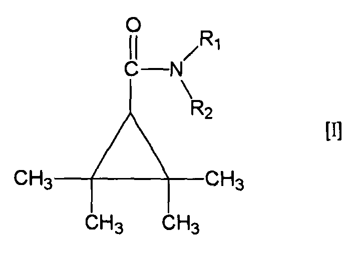 worin R₁ und R₂ unterschiedlich sind und eines von R₁ oder R₂ eine C₁-C₆ Alkylgruppe ist und das andere Wasserstoff ist, zur Verwendung in der Behandlung von bipolaren Störungen.

2. Die Verbindung nach Formel I gemäß Anspruch 1, worin die C₁-C₆ Alkylgruppe eine geradkettige oder eine verzweigte Alkylgruppe ist.

3. Die Verbindung der Formel I gemäß Anspruch 1, worin die C₁-C₆ Alkylgruppe eine Methylgruppe ist.

4. Die Verbindung der Formel I gemäß Anspruch 1, worin die Verbindung als eine pharmazeutische Zusammensetzung, umfassend eine Verbindung der Formel I und einen pharmazeutisch akzeptablen Träger, verabreicht wird.

5. Die Verbindung der Formel I gemäß Anspruch 1, worin der Verabreichungsweg der Verbindung ausgewählt ist aus der Gruppe bestehend aus oraler, parenteraler, topischer, perkutaner, rektaler und bukkaler Verabreichung.

6. Die Verbindung der Formel I gemäß Anspruch 1, worin der Verabreichungsweg der Verbindung ausgewählt ist aus der Gruppe bestehend aus oraler und parenteraler Verabreichung.

7. Die Verbindung der Formel I gemäß Anspruch 5 oder 6, worin der parenterale Verabreichungsweg ausgewählt ist aus der Gruppe bestehend aus intravenöser, intramuskulärer, intraperitonealer und subkutaner Verabreichung.

8. Die Verbindung der Formel I gemäß Anspruch 1, worin die Verbindung in einem Bereich von 1 mg bis 1000 mg pro Tag verabreicht wird.

9. Die Verbindung der Formel I gemäß Anspruch 1, worin die Verbindung in einem Bereich von 20 mg bis 500 mg pro Tag verabreicht wird.

## Revendications

1. Composé de formule I 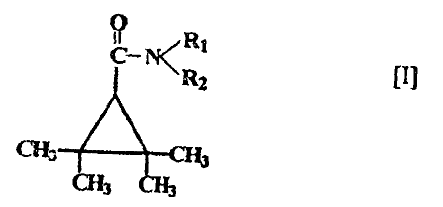 dans laquelle R₁ et R₂ sont différents et l'un parmi R₁ et R₂ est un groupe alkyle en C₁-C₆ et l'autre est un hydrogène, pour l'utilisation dans le traitement de troubles bipolaires.

2. Composé de formule I selon la revendication 1, dans lequel ledit groupe alkyle en C₁-C₆ est un groupe alkyle linéaire ou ramifié.

3. Composé de formule I selon la revendication 1, dans lequel ledit groupe alkyle en C₁-C₆ est un groupe méthyle.

4. Composé de formule I selon la revendication 1, dans lequel ledit composé est administré sous la forme d'une composition pharmaceutique comprenant un composé de formule I et un véhicule pharmaceutiquement acceptable.

5. Composé de formule I selon la revendication 1, dans lequel la voie d'administration dudit composé est choisie dans le groupe constitué par l'administration orale, parentérale, topique, transdermique, rectale et buccale.

6. Composé de formule I selon la revendication 1, dans lequel la voie d'administration dudit composé est choisie dans le groupe constitué par l'administration orale et parentérale.

7. Composé de formule I selon la revendication 5 ou 6, dans lequel ladite voie d'administration parentérale est choisie dans le groupe constitué par l'administration intraveineuse, intramusculaire, intrapéritonéale et sous-cutanée.

8. Composé de formule I selon la revendication 1, ledit composé étant administré dans la gamme de 1 mg à 1000 mg par jour.

9. Composé de formule I selon la revendication 1, ledit composé étant administré dans la gamme de 20 mg à 500 mg par jour.
